(19) Europäisches Patentamt
European Patent Office
Office européen des brevets

(11) Publication number : **0 286 153 B1**

# (12) EUROPEAN PATENT SPECIFICATION

(45) Date of publication of patent specification : **29.01.92 Bulletin 92/05**

(51) Int. Cl.$^5$ : **C07C 255/42,** C07D 233/26, C07D 239/06, A01N 37/34, A01N 43/50, A01N 43/54

(21) Application number : **88200463.3**

(22) Date of filing : **10.03.88**

## (54) Herbicidal vinylamine derivatives.

(30) Priority : **26.03.87 GB 8707320**

(43) Date of publication of application : **12.10.88 Bulletin 88/41**

(45) Publication of the grant of the patent : **29.01.92 Bulletin 92/05**

(84) Designated Contracting States : **AT BE CH DE ES FR GB GR IT LI LU NL SE**

(56) References cited :
**EP-A- 0 010 396**
**DE-A- 2 330 913**
**JOURNAL OF THE CHEMICAL SOCIETY, Perkin Transactions 1 Organic and Bio-organic chemistry, no. 12, 1981, pages 2997-3294; S. RAJAPPA et al.: "Unexpected substitution of the Acyl group in isothiazole-ring formation. Attempted conversion of 1-Acyl-2,2-diaminoethylenes into 2-Acyl-3,3-diaminoacrylonitriles"**

(73) Proprietor : **SHELL INTERNATIONALE RESEARCH MAATSCHAPPIJ B.V.**
**Carel van Bylandtlaan 30**
**NL-2596 HR Den Haag (NL)**

(72) Inventor : **Gilkerson, Terence**
**11, The Ness**
**Canterbury Kent (GB)**
Inventor : **Davis, Royston Henry**
**76, Bell Road**
**Sittingbourne Kent (GB)**
Inventor : **Shaw, Robert William**
**34 Berkely Court**
**Sittingbourne Kent (GB)**

(74) Representative : **Bennett, David Arthur Horder et al**
**4, York Road**
**London SE1 7NA (GB)**

EP 0 286 153 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to herbicidal compositions containing certain vinylamine derivatives and to a method of combatting undesired plant growth using such compositions. The invention also relates to novel vinylamine derivatives and to a process for their preparation.

J. Prakt. Chem. vol. 319 (4), pages 545-560 (1977), J. Chem. Soc. Perkin I (1981), pages 3162-63, and Tetrahedron vol. 36, pages 1791-1799 (1980) describe the preparation of various cyano-substituted vinylene derivatives. It has now been found that certain such compounds have useful herbicidal activity.

The present invention provides a herbicidal composition which comprises a carrier and, as active ingredient, a compound of the general formula I or a salt thereof:

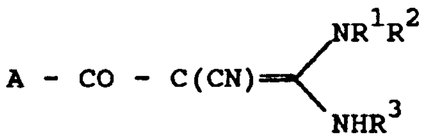

(I)

in which A represents a phenyl group optionally substituted by one or more of the same or different substituents selected from halogen atoms and nitro, cyano, amino, alkylamino, dialkylamino, hydroxy, phenoxy, alkyl, haloalkyl, alkylthio, alkylsulphonyl, alkoxycarbonyl, carboxy and alkoxy groups, and each of $R^1$, $R^2$, and $R^3$ independently represents a hydrogen atom, a cyclopropyl group, or an alkyl, alkenyl, alkynyl, alkoxy, alkenyloxy or alkynyloxy group optionally substituted by one or more of the same or different substituents selected from halogen atoms and alkoxy, alkylthio, amino, alkylamino and dialkylamino groups; the total number of carbon atoms present in each of $R^1$, $R^2$, or $R^3$ being not more than 6; or $R^1$ has one of the meanings given above, and $R^2$ and $R^3$ together represent a dimethylene or trimethylene group.

The substituent groups which may be present in an optionally-substituted phenyl group A are such as are customarily employed in the development of pesticidal compounds, and/or the modification of such compounds to influence their structure/activity, persistence, penetration or other property. Typically from 1 to 3 substituents may be employed. Halogen atoms are preferably fluorine, chlorine or bromine. Any alkyl moiety present in a phenyl substituent preferably has up to 4, especially 1 or 2, carbon atoms.

Especially preferred groups A are phenyl groups mono-substituted, preferably in the 4-position, or disubstituted, preferably in the 3,4-positions, by chlorine and/or fluorine atoms.

As stated above, the total number of carbon atoms present in each of $R^1$, $R^2$ or $R^3$ is not more than 6. Preferably any individual alkyl, alkenyl or alkynyl moiety has up to 4, especially up to 2, carbon atoms. Preferred substituents of a substituted alkyl, alkenyl, alkynyl, alkoxy, alkenyloxy or alkynyloxy group $R^1$, $R^2$ or $R^3$ are halogen atoms and alkoxy, alkylamino and dialkylamino groups in which the or each alkyl moiety is an ethyl or, especially, methyl, group.

Preferably $R^1$ represents a hydrogen atom.

Preferably each of $R^2$ and $R^3$ independently represents a cyclopropyl group or an alkyl, alkenyl, alkynyl, alkoxy, alkenyloxy or alkynyloxy group having up to 4 carbon atoms, optionally substituted by a halogen, methoxy, methylamino or dimethylamino moiety, or $R^2$ and $R^3$ together represent a dimethylene group. Especially preferred are compounds in which each of $R^2$ and $R^3$ independently represents an unsubstituted alkyl group having 1 to 4, especially 2 or 3, carbon atoms.

The compounds of the general formula I form acid addition salts. Suitable salts include those with mineral acids, for example hydrohalic acids, and organic acids such as acetic, benzoic, tartaric, oxalic and citric acid.

It will be appieciated that compounds of formula I exist in isomeric form; isomers fall within the scope of this invention. As is well known, it is frequently the case that one isomer will have greater biological activity than another isomer.

Some of the compounds of the general formula I and their salts are novel, and accordingly the invention provides a compound of the general formula I or a salt thereof, in which A, $R^1$, $R^2$, and $R^3$ have the meanings given above with the provisos that (i) if the phenyl group A is mono-substituted in the 4-position by chlorine, bromine or methoxy, or di-substituted in the 3,4-positions by chlorine, and $R^2$ and $R^3$ together represent dimethylene, then $R^1$ is other than hydrogen; (ii) if the phenyl group A is unsubstituted or mono-substituted in the 4-position by chlorine or methyl, and $R^2$ and $R^3$ together represent trimethylene, then $R^1$ is other than methyl; and (iii) if $R^1$ is hydrogen and $R^2$ and $R^3$ are the same and are hydrogen, methyl or ethyl or if $R^1$ is hydrogen or methyl and $R^2$ and $R^3$ together are dimethylene, then the phenyl group A must carry at least one substituent.

The compounds of the formula I and their salts may be prepared by reaction of the appropriate bis-alkylthio compound with the appropriate amine or amines. If the groups $NR^1R^2$ and $NHR^3$ are the same, or if $R^2$ and $R^3$

together represent a di- or tri-methylene group, both alkylthio groups can be replaced with amine groups in a single step. If $NR^1R^2$ and $NHR^3$ are different, the reaction may be carried out by replacing one alkylthio group with a first amine, and subsequently replacing the second alkylthio group with a second amine.

Accordingly the invention provides a process for the preparation of a novel compound according to the invention, which comprises reacting a compound of the general formula

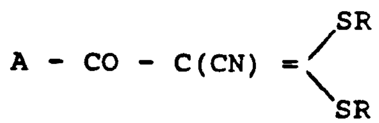

(II)

in which each R represents an alkyl group and A has the meaning given for the general formula I, with a compound of the general formula $NHR^1R^2$, optionally followed by reaction with a compound of the general formula $NH_2R^3$; or with a compound of the general formula $NHR^1(CH_2)_nNH_2$ in which n is 2 or 3.

The process of the invention is preferably carried out in the presence of a suitable solvent, for example an ether, ester or alcohol. Preferably the reaction temperature is in the range of from -20 to 50°C, especially -10 to 20°C. If the groups $NR^1R^2$ and $NHR^3$ are the same, or if $R^2$ and $R^3$ together represent a di- or tri-methylene group, the reaction is suitably carried out in one step by reacting each mole of the compound of the formula II with at least two moles of amine $HNR^1R^2$ or at least one mole of amine $NHR^1(CH_2)_nNH_2$. If the groups $NR^1R^2$ and $NHR^3$ are different, yields are maximised by reacting each mole of the compound of the general formula II with one mole of amine $NNR^1R^2$, allowing this reaction to go to completion, and then reacting with at least one mole of amine $NH_2R^3$.

Preferably in the compound of the general formula II, each R represents a methyl group. The compounds of formula II are known, and can be prepared by known methods.

The compounds of general formula I have been found to show interesting activity as herbicides. Accordingly, the invention further provides a herbicidal composition comprising a compound of formula I as defined above in association with at least one carrier, and a method of making such a composition which comprises bringing a compound of formula I into association with at least one carrier.

The invention also provides the use of such a compound or composition according to the invention as a herbicide. Further, in accordance with the invention there is provided a method of combating undesired plant growth at a locus by treating the locus with a compound or composition according to the invention. Application to the locus may be pre-emergence or post-emergence. The dosage of active ingredient used may, for example, be from 0.01 to 10 kg/ha, suitably 0.05 to 4kg/ha. The locus may for example be a crop area, typical crops being cereals such as wheat, barley and rice. A wide range of weeds is controlled, particularly broad-leaved weeds, including Gallium, often regarded as a particular problem in these crops. A carrier in a composition according to the invention is any material with which the active ingredient is formulated to facilitate application to the locus to be treated which may for example be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montomorillonites and micas; calcium carbonate, calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing $\frac{1}{2}$-10% w of active ingredient. Granules are usually prepared to have a size between 10 and 100 BS mesh (1.676 - 0.152 mm), and may be manufactured by agglomeration or impregnation techniques. Generally, granules will contain $\frac{1}{2}$-75% w active ingredient and 0-10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'-like consistency.

The composition of the invention may also contain other ingredients, for example other compounds possessing herbicidal, insecticidal or fungicidal properties.

The following Examples illustrate the invention.

Example 1

Preparation of 1-(4-Chlorobenzoyl)-1-cyano-2-ethylamino-2n-propylamino ethene.

N-Propylamine (2ml) was added dropwise to a stirred, ice-cold suspension of 1-(4-chlorobenzoyl)-1-cyano-2,2-bis-(methylthio)ethene (7g) in dry diethyl ether (100ml). After stirring for a further 3 hours at room temperature, the mixture was evaporated and the residue purified by flash column chromatography using 2.5% $MeOH-CH_2Cl_2$ as eluant. Recrystallisation from ethanol gave 1-(4-Chlorobenzoyl)-1-cyano-2-methylthio-2-n-propylamino ethene as a yellow solid (3.5g, 50%) of melting point 94-96°C.

Ethylamine (0.4g) was added to a stirred suspension of this product (2g) in dry diethyl ether. After stirring at room temperature for 3 hours, the product was filtered and washed with diethyl ether. Recrystallisation from ethanol gave the title compound as a white solid (1g, 50%) of melting point 106-108°C.

| Elemental Analysis | C | H | N |
|---|---|---|---|
| Calculated: | 61.7 | 6.2 | 14.4 |
| Found : | 61.8 | 6.2 | 14.4 |

Example 2

Preparation of 1-(4-Chlorobenzoyl)-1-cyano-2,2-bis(ethylamino)-ethene.

Ethylamine (4ml) was added dropwise to a stirred, ice-cold, suspension of 1-(4-chlorobenzoyl)-1-cyano-2,2-bis-(methylthio)ethene (2.8g) in dry diethyl ether (50ml). After stirring for a further 3 hours at room temperature, the product was filtered off and recrystallised from ethanol to give the title compound (2.0g, 73.5%) of melting point 149-150°.

| Elemental Analysis | C | H | N |
|---|---|---|---|
| Calculated: | 60.5 | 6.2 | 15.1 |
| Found : | 60.6 | 6.2 | 15.1 |

Examples 3 to 79

By methods analogous to those described in Examples 1 and 2, further compounds of the general formula I were prepared. Details are given in Table 1.

TABLE 1

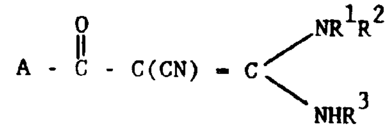

| Example No. | Substituents in Phenyl Group A | $R^1$ | $R^2$ | $R^3$ | Elemental Analysis 1st row calculated values 2nd row found values C | H | N | Melting Point °C |
|---|---|---|---|---|---|---|---|---|
| 3 | - | H | Et | Et | 69.1 | 7.0 | 17.3 | 126-127 |
| | | | | | 69.1 | 7.0 | 17.3 | |
| 4 | - | H | Me | Me | 66.9 | 6.0 | 19.5 | 154-155 |
| | | | | | 67.0 | 6.0 | 19.5 | |
| 5 | 2-Cl | H | Et | Et | 60.5 | 6.2 | 15.1 | 151-153 |
| | | | | | 60.7 | 5.8 | 15.3 | |

Table 1 (continued)

| Example No. | Substituents in Phenyl Group A | $R^1$ | $R^2$ | $R^3$ | Elemental Analysis 1st row calculated values 2nd row found values C | H | N | Melting Point °C |
|---|---|---|---|---|---|---|---|---|
| 6 | - | H | $-(CH_2-CH_2)-$ | | 67.6 | 5.1 | 19.7 | 217-220 |
| | | | | | 67.3 | 5.2 | 19.6 | |
| 7 | 4-Cl | H | Me | Me | 57.7 | 4.8 | 16.8 | 215-217 |
| | | | | | 57.6 | 4.9 | 17.0 | |
| 8 | 4-Cl | H | $-(CH_2-CH_2)-$ | | 58.1 | 4.0 | 15.9 | 221-223 |
| | | | | | 58.0 | 4.0 | 16.2 | |
| 9 | 4-F | H | Me | Me | 62.0 | 5.2 | 18.1 | 195-197 |
| | | | | | 61.8 | 5.3 | 17.8 | |

Table 1 (continued)

| Example No. | Substituents in Phenyl Group A | $R^1$ | $R^2$ | $R^3$ | Elemental Analysis 1st row calculated values 2nd row found values C | H | N | Melting Point °C |
|---|---|---|---|---|---|---|---|---|
| 10 | 4-F | H | Et | Et | 64.4 | 6.1 | 16.1 | 130-132 |
| | | | | | 64.3 | 6.1 | 16.1 | |
| 11 | 4-F | H | $-(CH_2-CH_2)-$ | | 62.3 | 4.3 | 18.2 | 210-212 |
| | | | | | 62.1 | 4.4 | 18.1 | |
| 12 | 3-F | H | Me | Me | 62.0 | 5.2 | 18.1 | 154-155 |
| | | | | | 62.0 | 5.4 | 18.0 | |
| 13 | 3-F | H | Et | Et | 64.4 | 6.1 | 16.1 | 123-125 |
| | | | | | 64.4 | 6.3 | 16.1 | |

Table 1 (continued)

| Example No. | Substituents in Phenyl Group A | $R^1$ | $R^2$ | $R^3$ | Elemental Analysis 1st row calculated values 2nd row found values C | H | N | Melting Point °C |
|---|---|---|---|---|---|---|---|---|
| 14 | 3-F | H | $-(CH_2-CH_2)-$ | | 62.3 | 4.3 | 18.2 | 193-194 |
| | | | | | 62.0 | 4.4 | 18.1 | |
| 15 | 2-Cl | H | $-(CH_2-CH_2)-$ | | 58.1 | 4.0 | 16.9 | 231-233 |
| | | | | | 58.4 | 4.2 | 16.9 | |
| 16 | $3-CF_3$ | H | Me | Me | 55.1 | 4.2 | 14.8 | 154-155 |
| | | | | | 55.1 | 4.1 | 14.6 | |
| 17 | $3-CF_3$ | H | Et | Et | 57.8 | 5.1 | 13.5 | 123-124 |
| | | | | | 57.6 | 5.2 | 13.7 | |

Table 1 (continued)

| Example No. | Substituents in Phenyl Group A | $R^1$ | $R^2$ | $R^3$ | Elemental Analysis 1st row calculated values 2nd row found values C | H | N | Melting Point °C |
|---|---|---|---|---|---|---|---|---|
| 18 | 3-$CF_3$ | H | -$(CH_2$-$CH_2)$- | | 55.5 | 3.5 | 14.9 | 180-182 |
| | | | | | 55.2 | 3.5 | 14.9 | |
| 19 | 4-Me | H | Me | Me | 68.1 | 6.5 | 18.3 | 189-191 |
| | | | | | 67.7 | 6.4 | 17.1 | |
| 20 | 4-Me | H | Et | Et | 70.0 | 7.4 | 16.3 | 154-155 |
| | | | | | 70.3 | 7.4 | 16.3 | |
| 21 | 3-Me | H | Me | Me | 68.1 | 6.5 | 18.3 | 140-141 |
| | | | | | 68.1 | 6.6 | 18.4 | |

Table 1 (continued)

| Example No. | Substituents in Phenyl Group A | $R^1$ | $R^2$ | $R^3$ | Elemental Analysis 1st row calculated values 2nd row found values C | H | N | Melting Point °C |
|---|---|---|---|---|---|---|---|---|
| 22 | 3-Me | H | Et | Et | 70.0 | 7.4 | 16.3 | 126-127 |
| | | | | | 70.0 | 7.5 | 16.3 | |
| 23 | 4-OMe | H | Me | Me | 63.6 | 6.1 | 17.1 | 174-176 |
| | | | | | 63.6 | 6.2 | 17.1 | |
| 24 | 4-OMe | H | Et | Et | 65.9 | 6.9 | 15.4 | 155-156 |
| | | | | | 65.8 | 7.0 | 15.2 | |
| 25 | 3-OMe | H | Me | Me | 63.6 | 6.1 | 17.1 | 145-146 |
| | | | | | 64.2 | 6.1 | 17.0 | |

Table 1 (continued)

| Example No. | Substituents in Phenyl Group A | $R^1$ | $R^2$ | $R^3$ | Elemental Analysis 1st row calculated values 2nd row found values C | H | N | Melting Point °C |
|---|---|---|---|---|---|---|---|---|
| 26 | 3-OMe | H | Et | Et | 65.9 | 6.9 | 15.4 | 144-145 |
| | | | | | 66.3 | 7.0 | 15.3 | |
| 27 | 2-OMe | H | Et | Et | 65.9 | 6.9 | 15.4 | 130-131 |
| | | | | | 65.8 | 7.1 | 15.4 | |
| 28 | 4-CN | H | Et | Et | 67.1 | 5.9 | 20.9 | 153-154 |
| | | | | | 66.7 | 6.0 | 20.7 | |
| 29 | 4-$^i$Pr | H | Me | Me | 70.0 | 7.4 | 16.3 | 128-129 |
| | | | | | 69.5 | 7.4 | 16.2 | |

Table 1 (continued)

| Example No. | Substituents in Phenyl Group A | $R^1$ | $R^2$ | $R^3$ | Elemental Analysis 1st row calculated values 2nd row found values C | H | N | Melting Point °C |
|---|---|---|---|---|---|---|---|---|
| 30 | 3,4-di-F | H | Me | Me | 57.3 | 4.4 | 16.7 | 164-166 |
| | | | | | 57.3 | 4.5 | 16.3 | |
| 31 | 3,4-di-F | H | Et | Et | 60.2 | 5.4 | 15.0 | 121-123 |
| | | | | | 60.1 | 5.4 | 15.0 | |
| 32 | 3,4-di-Me | H | Et | Et | 70.8 | 7.7 | 15.5 | 151-152 |
| | | | | | 70.8 | 7.8 | 15.5 | |
| 33 | 4-Cl | H | $^n$Pr | $^n$Pr | 62.8 | 6.5 | 13.7 | 120-121 |
| | | | | | 62.9 | 6.5 | 13.7 | |

Table 1 (continued)

| Example No. | Substituents in Phenyl Group A | $R^1$ | $R^2$ | $R^3$ | Elemental Analysis 1st row calculated values 2nd row found values C | H | N | Melting Point °C |
|---|---|---|---|---|---|---|---|---|
| 34 | 4-Cl | H | $^nBu$ | $^nBu$ | 64.7 | 7.2 | 12.6 | 74-76 |
| | | | | | 65.1 | 7.0 | 12.6 | |
| 35 | 4-Cl | H | $(CH_2)_4Me$ | $(CH_2)_4Me$ | 66.4 | 7.7 | 11.6 | oil |
| | | | | | 64.4 | 7.7 | 11.0 | |
| 36 | 4-F | H | $^nPr$ | $^nPr$ | 66.4 | 6.9 | 14.5 | 109-110 |
| | | | | | 66.3 | 7.1 | 14.4 | |
| 37 | 4-Cl | H | $^nPr$ | Me | 60.5 | 5.8 | 15.1 | 133-134 |
| | | | | | 60.4 | 5.9 | 15.1 | |

Table 1 (continued)

| Example No. | Substituents in Phenyl Group A | $R^1$ | $R^2$ | $R^3$ | Elemental Analysis 1st row calculated values 2nd row found values C | H | N | Melting Point °C |
|---|---|---|---|---|---|---|---|---|
| 38 | 4-Cl | H | allyl | allyl | 63.6 | 51.3 | 13.9 | 108-110 |
| | | | | | 63.7 | 5.3 | 13.9 | |
| 39 | 4-Cl | H | Et | $^i$Bu | 62.8 | 6.5 | 13.7 | 113-114 |
| | | | | | 62.7 | 6.6 | 13.6 | |
| 40 | 4-Cl | H | Et | $^i$Pr | 61.7 | 6.1 | 14.4 | 113-115 |
| | | | | | 61.1 | 6.0 | 14.1 | |
| 41 | 4-Cl | H | Et | $^n$Bu | 62.8 | 6.5 | 13.7 | 93-94 |
| | | | | | 62.7 | 6.5 | 13.5 | |

Table 1 (continued)

| Example No. | Substituents in Phenyl Group A | $R^1$ | $R^2$ | $R^3$ | Elemental Analysis 1st row calculated values 2nd row found values C | H | N | Melting Point °C |
|---|---|---|---|---|---|---|---|---|
| 42 | 4-Cl | H | Et | allyl | 62.1 | 5.5 | 14.5 | 112-114 |
| | | | | | 61.9 | 5.5 | 14.3 | |
| 43 | 4-Cl | Me | Me | Et | 60.5 | 5.7 | 15.1 | 164-166 |
| | | | | | 60.4 | 5.7 | 15.1 | |
| 44 | 4-Cl | H | cyclopropyl | cyclopropyl | 63.6 | 5.3 | 13.9 | 127-128 |
| | | | | | 63.6 | 5.3 | 13.9 | |
| 45 | 4-Cl | H | $^{n}Pr$ | $^{n}Bu$ | 63.8 | 6.9 | 13.1 | 78-79 |
| | | | | | 63.8 | 6.9 | 13.2 | |

Table 1 (continued)

| Example No. | Substituents in Phenyl Group A | $R^1$ | $R^2$ | $R^3$ | Elemental Analysis 1st row calculated values 2nd row found values C | H | N | Melting Point °C |
|---|---|---|---|---|---|---|---|---|
| 46 | 4-Cl | H | Me | $^{i}Bu$ | 61.7<br>61.7 | 6.1<br>6.1 | 14.4<br>14.3 | 115-117 |
| 47 | 4-Cl | H | Me | $^{n}Bu$ | 61.7<br>61.7 | 6.1<br>6.2 | 14.4<br>14.4 | 120-122 |
| 48 | 4-Cl | H | $^{i}Bu$ | $^{i}Bu$ | 64.7<br>64.8 | 7.2<br>7.2 | 12.6<br>12.6 | 118-120 |
| 49 | 4-Cl | H | $^{n}Pr$ | $^{i}Bu$ | 62.8<br>62.7 | 6.5<br>6.5 | 13.7<br>13.6 | 127-129 |

Table 1 (continued)

| Example No. | Substituents in Phenyl Group A | $R^1$ | $R^2$ | $R^3$ | Elemental Analysis 1st row calculated values 2nd row found values C | H | N | Melting Point °C |
|---|---|---|---|---|---|---|---|---|
| 50 | 4-Cl | H | Et | Me | 59.2 | 5.3 | 15.9 | 195-196 |
| | | | | | 59.2 | 5.4 | 15.9 | |
| 51 | 4-Cl | H | Me | $CH_2CH=CH_2$ | 60.9 | 5.1 | 15.2 | 154-156 |
| | | | | | 60.9 | 5.1 | 15.1 | |
| 52 | 4-Cl | H | $^nPr$ | $CH_2CH=CH_2$ | 63.2 | 5.9 | 13.8 | 100-102 |
| | | | | | 63.3 | 5.9 | 13.8 | |
| 53 | 4-Cl | H | $^nPr$ | $^iPr$ | 62.8 | 6.5 | 13.7 | 136-138 |
| | | | | | 62.7 | 6.5 | 13.6 | |

Table 1 (continued)

| Example No. | Substituents in Phenyl Group A | $R^1$ | $R^2$ | $R^3$ | Elemental Analysis 1st row calculated values 2nd row found values C | H | N | Melting Point °C |
|---|---|---|---|---|---|---|---|---|
| 54 | 4-Cl | H | $^nPr$ | $CH_2C{\equiv}CH$ | 63.6 | 5.3 | 13.9 | 112-114 |
| | | | | | 63.8 | 5.0 | 14.0 | |
| 55 | 4-Cl | H | Et | $-OCH_2CH{=}CH_2$ | 68.9 | 5.2 | 13.7 | 84-85 |
| | | | | | 68.9 | 5.4 | 14.0 | |
| 56 | 4-Cl | H | Et | $^sBu$ | 62.8 | 6.5 | 13.7 | 105-106 |
| | | | | | 63.1 | 6.5 | 13.7 | |
| 57 | 4-Cl | H | $^nPr$ | H | 59.2 | 5.3 | 15.9 | 122-124 |
| | | | | | 58.6 | 5.2 | 15.5 | |

Table 1 (continued)

| Example No. | Substituents in Phenyl Group A | $R^1$ | $R^2$ | $R^3$ | Elemental Analysis 1st row calculated values 2nd row found values C | H | N | Melting Point °C |
|---|---|---|---|---|---|---|---|---|
| 58 | 4-Cl | H | Et | OEt | 57.2 | 5.4 | 14.3 | 98-99 |
| | | | | | 56.9 | 5.5 | 14.7 | |
| 59 | 4-Cl | H | Et | $CH_2CH_2Cl$ | 54.4 | 4.2 | 13.6 | 161-162 |
| | | | | | 55.0 | 4.3 | 13.5 | |
| 60 | 4-Cl | H | Et | $(CH_2)_3Cl$ | 55.2 | 5.2 | 12.8 | 86-87 |
| | | | | | 53.9 | 5.1 | 11.9 | |
| 61 | 4-F | H | $^nPr$ | Et | 65.4 | 6.5 | 15.3 | 113-115 |
| | | | | | 65.4 | 6.4 | 15.0 | |

Table 1 (continued)

| Example No. | Substituents in Phenyl Group A | $R^1$ | $R^2$ | $R^3$ | Elemental Analysis 1st row calculated values 2nd row found values C | H | N | Melting Point °C |
|---|---|---|---|---|---|---|---|---|
| 62 | 3,4-di-Cl | H | Et | Et | 53.8 | 4.8 | 13.5 | 132-133 |
| | | | | | 53.6 | 4.8 | 13.4 | |
| 63 | 3,4-di-Cl | H | $^n$Pr | $^n$Pr | 56.5 | 5.6 | 12.3 | 78-80 |
| | | | | | 56.0 | 5.6 | 12.3 | |
| 64 | 4-Cl | H | $^n$Pr | $CH_2CF_3$ | 52.1 | 4.3 | 12.2 | 128-130 |
| | | | | | 54.8 | 4.6 | 12.2 | |
| 65 | 4-Et | H | Et | Et | 70.8 | 7.7 | 15.5 | 131-133 |
| | | | | | 69.9 | 7.7 | 14.9 | |

Table 1 (continued)

| Example No. | Substituents in Phenyl Group A | $R^1$ | $R^2$ | $R^3$ | Elemental Analysis 1st row calculated values 2nd row found values C | H | N | Melting Point °C |
|---|---|---|---|---|---|---|---|---|
| 66 | 4-$NMe_2$ | H | $^nPr$ | $^nPr$ | 68.7 | 8.3 | 17.8 | 138-140 |
| | | | | | 68.7 | 8.3 | 17.7 | |
| 67 | 4-Et | H | $^nPr$ | $^nPr$ | 72.7 | 7.7 | 14.1 | 121-123 |
| | | | | | 72.8 | 8.4 | 14.0 | |
| 68 | 4-S(O)Et | H | $^nPr$ | $^nPr$ | 62.2 | 7.2 | 12.1 | oil |
| | | | | | 58.5 | 6.4 | 11.8 | |
| 69 | 3-phenoxy | H | Et | Et | 71.6 | 6.3 | 12.5 | 98-100 |
| | | | | | 71.7 | 6.3 | 12.5 | |

Table 1 (continued)

| Example No. | Substituents in Phenyl Group A | $R^1$ | $R^2$ | $R^3$ | Elemental Analysis 1st row calculated values 2nd row found values C | H | N | Melting Point °C |
|---|---|---|---|---|---|---|---|---|
| 70 | 3-phenoxy | H | $^nPr$ | $^nPr$ | 72.7<br>71.8 | 6.8<br>6.9 | 11.5<br>11.5 | 47-50 |
| 71 | 4-Cl | H | $CH_2C\equiv CH$ | $CH_2C\equiv CH$ | 64.5<br>64.1 | 4.0<br>4.1 | 14.1<br>13.9 | 125-126 |
| 72 | 4-Cl | H | $(CH_2)_2NMe_2$ | Et | 59.9<br>60.0 | 6.5<br>6.6 | 17.4<br>17.4 | 74-75 |
| 73 | 4-Cl | H | $(CH_2)_2NMe_2$ | $^nPr$ | 61.0<br>61.5 | 6.9<br>7.0 | 16.7<br>17.1 | 75-76 |

Table 1 (continued)

| Example No. | Substituents in Phenyl Group A | $R^1$ | $R^2$ | $R^3$ | Elemental Analysis 1st row calculated values 2nd row found values C | H | N | Melting Point °C |
|---|---|---|---|---|---|---|---|---|
| 74 | 4-Cl | H | $(CH_2)_2NMe_2$ | Me | 58.7 | 6.2 | 18.3 | 95-96 |
| | | | | | 58.8 | 6.3 | 18.3 | |
| 75 | 4-Cl | H | $(CH_2)_2OMe$ | Et | 58.5 | 5.8 | 13.6 | 68-69 |
| | | | | | 57.7 | 6.0 | 13.3 | |
| 76 | 4-Cl | H | $(CH_2)_2OMe$ | $^nPr$ | 59.7 | 6.2 | 13.0 | 64-65 |
| | | | | | 58.7 | 6.2 | 12.9 | |
| 77 | 4-Cl | H | $(CH_2)_2OMe$ | Me | 57.2 | 5.4 | 14.3 | 83-84 |
| | | | | | 56.4 | 5.4 | 14.2 | |

Table 1 (continued)

| Example No. | Substituents in Phenyl Group A | $R^1$ | $R^2$ | $R^3$ | Elemental Analysis 1st row calculated values 2nd row found values C | H | N | Melting Point °C |
|---|---|---|---|---|---|---|---|---|
| 78 | 3-Cl | H | Et | Et | 60.5 | 5.8 | 15.1 | 120-122 |
| | | | | | 60.7 | 5.8 | 15.1 | |
| 79 | 3-Cl | H | $^nPr$ | $^nPr$ | 62.8 | 6.6 | 13.7 | 71-73 |
| | | | | | 63.1 | 6.6 | 13.6 | |

Example 80

Preparation of 1-(4-Fluorobenzoyl)-1-cyano-2,2-bis(ethylamino)ethene hydrochloride

Dry hydrochloric acid gas was passed through a stirred suspension of 1-(4-fluorobenzoyl)-1-cyano-2,2-bis(ethylamino) ethene (1.0g) in dry diethyl ether (50 ml) until no further visible change in the formation of the amorphous white hydrochloride salt could be observed (about 15 mins.). Filtration gave the title compound (1.0g) of m.p. 162-164°C.

Analysis:

| | |
|---|---|
| Calc. for $C_{14}H_{17}N_3OFCl$: | C 56.5; H 5.7; N 14.1% |
| Found: | C 56.7; H 5.8; N 14.1% |

Example 81

Preparation of 1-(4-chlorobenzoyl)-1-cyano-2,2-bis(n-propyl- amino)ethene oxalate

An excess of a saturated solution of oxalic acid in diethyl ether (20ml) was added to a stirred suspension of 1-(4-chlorobenzoyl)-1-cyano-2,2-bis(n-propylamino)ethene (1g) in diethyl ether (50ml) at room temperature. After stirring for a further 1 hour, the white solid was filtered and washed with diethyl ether to give the title compound (0.8g) of m.p. 141-142°C.

Examples 82 to 84

By methods analogous to those described in Examples 80 and 81, further salts according to the invention were prepared. Details are given in Table 2.

TABLE 2

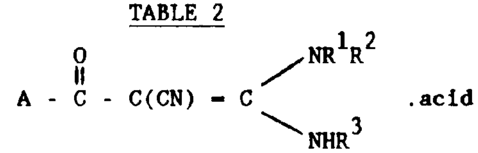

| Example No. | Acid | Substituents in Phenyl Group A | $R^1$ | $R^2$ | $R^3$ | Elemental Analysis 1st row calculated values 2nd row found values C | H | N | Melting Point °C |
|---|---|---|---|---|---|---|---|---|---|
| 82 | HCl | 4-Cl | H | $^n$Pr | $^n$Pr | 55.9 | 6.1 | 12.3 | 156-158 |
| | | | | | | 56.1 | 6.2 | 12.8 | |
| 83 | HCl | 4-Et | H | $^n$Pr | $^n$Pr | 64.4 | 7.7 | 12.5 | 165-166 |
| | | | | | | 66.2 | 8.1 | 12.6 | |
| 84 | HCl | 3,4-di-Cl | H | Et | Et | 48.0 | 4.6 | 12.0 | 158-161 |
| | | | | | | 48.6 | 4.6 | 11.9 | |

Example 85

Herbicidal Activity

To evaluate their herbicidal activity, compounds according to the invention were tested using as representative range of plants: maize, Zea mays (Mz); rice, Oryza sativa (R); barnyard grass, Echinochloa crusgalli (BG); oat, Avena sativa (O); linseed, Linum usitatissisum (L); mustard, Sinapsis alba (M); sugar beet, Beta vulgaris (SB) and soya bean, Glycine max (S).

The tests fall into two categories, pre-emergence and post-emergence. The pre-emergence tests involved spraying a liquid formulation of the compound onto the soil in which the seeds of the plant species mentioned above had recently been sown. The post-emergence tests involved two types of test, viz., soil drench and foliar spray tests. In the soil drench tests the soil in which the seedling plants of the above species were growing was drenched with a liquid formulation containing a compound of the invention, and in the foliar spray tests the seedling plants were sprayed with such a formulation.

The soil used in the tests was a prepared horticultural loam.

The formulations used in the tests were prepared from solutions of the test compounds in acetone containing 0.4% by weight of an alkylphenol/ethylene oxide condensate available under the trade mark TRITON X-155. These acetone solutions were diluted with water and the resulting formulations applied at dosage levels corresponding to 5 kg or 1 kg of active material per hectare in a volume equivalent to 900 litres per hectare in the soil spray and foliar spray test, and at a dosage of level equivalent to 10 kilograms of active material per hectare in a volume equivalent to approximately 3,000 litres per hectare in the soil drench tests.

In the pre-emergence tests untreated sown soil and in the post-emergence tests untreated soil bearing seedling plants were used as controls.

The herbicidal effects of the test compounds were assessed visually twelve days after spraying the foliage and the soil, and thirteen days after drenching the soil and were recorded on a 0-9 scale. A rating 0 indicates growth as untreated control, a rating 9 indicates death. An increase of 1 unit on the linear scale approximates to a 10% increase in the level of effect. The symbol '-' indicates that testing was not effected.

The results of the tests are set out in Table 3 below.

TABLE 3

| Compound of Ex. No. | Soil drench 10/kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 1 | 6 | 5 | 6 | 3 | 2 | 5 | 7 | 5 | 5 | 3 | 3 | 5 | 1 | 2 | 5 | 6 | 5 | 6 | 4 | 7 | 2 | 0 | 5 | 9 | 3 |
| | | | | | | | | | 1 | 2 | 0 | 0 | 0 | 2 | 3 | 4 | 5 | 4 | 2 | 6 | 1 | 0 | 1 | 9 | 0 |
| 2 | 6 | 3 | 6 | 5 | 4 | 5 | 6 | 5 | 5 | 3 | 2 | 5 | 5 | 2 | 4 | 5 | 4 | 7 | 5 | 7 | 5 | 6 | 8 | 9 | 5 |
| | | | | | | | | | 1 | 1 | 0 | 2 | 2 | 1 | 3 | 4 | 4 | 6 | 3 | 5 | 3 | 3 | 5 | 9 | 1 |
| 3 | 2 | 2 | 2 | 3 | 3 | 5 | 5 | 6 | 5 | 2 | 0 | 2 | 0 | 2 | 1 | 6 | 4 | 3 | 0 | 2 | 3 | 2 | 5 | 8 | 4 |
| | | | | | | | | | 1 | 1 | 0 | 2 | 0 | 2 | 1 | 4 | 2 | 2 | 0 | 2 | 0 | 0 | 2 | 5 | 2 |
| 4 | 0 | 3 | 5 | 5 | 6 | 6 | 9 | 5 | 5 | 0 | 0 | 3 | 2 | 6 | 6 | 8 | 4 | 0 | 0 | 5 | 4 | 4 | 7 | 7 | 3 |
| | | | | | | | | | 1 | 0 | 0 | 0 | 1 | 4 | 3 | 6 | 2 | 0 | 0 | 0 | 0 | 1 | 4 | 4 | 0 |

TABLE 3 (continued)

| Compound of Ex. No. | Soil drench 10/kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 5 | 0 | 0 | 0 | 2 | 0 | 3 | 6 | 5 | 5 | 3 | 0 | 2 | 2 | 3 | 2 | 5 | 5 | 0 | 0 | 3 | 2 | 0 | 5 | 8 | 5 |
| | | | | | | | | | 1 | 2 | 0 | 2 | 1 | 2 | 1 | 2 | 4 | 0 | 0 | 0 | 0 | 0 | 5 | 6 | 3 |
| 6 | 3 | 0 | 2 | 2 | 3 | 4 | 6 | 3 | 5 | 0 | 0 | 0 | 0 | 0 | 0 | 0 | 0 | 0 | 0 | 0 | 0 | 0 | 0 | 0 | 0 |
| | | | | | | | | | 1 | 0 | 0 | 0 | 0 | 0 | 0 | 0 | 0 | 0 | 0 | 0 | 0 | 0 | 0 | 0 | 0 |
| 7 | 0 | 0 | 2 | 3 | 2 | 4 | 5 | 2 | 5 | 0 | 0 | 0 | 0 | 0 | 0 | 0 | 0 | 0 | 0 | 3 | 2 | 3 | 5 | 3 | 2 |
| | | | | | | | | | 1 | 0 | 0 | 0 | 0 | 0 | 0 | 0 | 0 | 0 | 0 | 0 | 0 | 0 | 0 | 0 | 0 |
| 8 | 2 | 0 | 5 | 3 | 0 | 2 | 5 | 5 | 5 | 0 | 0 | 0 | 0 | 0 | 2 | 3 | 2 | 0 | 0 | 5 | 1 | 0 | 2 | 5 | 0 |
| | | | | | | | | | 1 | 0 | 0 | 0 | 0 | 0 | 0 | 1 | 1 | 0 | 0 | 0 | 0 | 0 | 2 | 4 | 0 |

TABLE 3 (continued)

| Compound of | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 9 | 4 | 3 | 3 | 4 | 5 | 6 | 7 | 5 | 5 | 1 | 1 | 2 | 1 | 3 | 5 | 6 | 4 | 0 | 0 | 4 | 4 | 5 | 7 | 7 | 3 |
| | | | | | | | | | 1 | 1 | 0 | 0 | 0 | 3 | 3 | 4 | 3 | 0 | 0 | 2 | 1 | 2 | 5 | 5 | 0 |
| 10 | 7 | 4 | 6 | 5 | 4 | 5 | 7 | 7 | 5 | 3 | 2 | 4 | 2 | 2 | 5 | 7 | 6 | 7 | 4 | 7 | 5 | 1 | 7 | 9 | 7 |
| | | | | | | | | | 1 | 1 | 1 | 2 | 1 | 2 | 3 | 4 | 5 | 4 | 2 | 5 | 2 | 0 | 3 | 9 | 4 |
| 11 | 0 | 2 | 0 | 1 | 4 | 3 | 3 | 5 | 5 | 0 | 2 | 2 | 0 | 2 | 3 | 4 | 5 | 0 | 0 | 0 | 0 | 2 | 5 | 3 | 3 |
| | | | | | | | | | 1 | 0 | 2 | 1 | 0 | 2 | 1 | 2 | 3 | 0 | 0 | 0 | 0 | 0 | 1 | 0 | 0 |
| 12 | 0 | 2 | 3 | 5 | 6 | 5 | 7 | 4 | 5 | 0 | 0 | 2 | 1 | 5 | 6 | 7 | 5 | 0 | 0 | 0 | 0 | 5 | 6 | 4 | 0 |
| | | | | | | | | | 1 | 0 | 0 | 0 | 0 | 3 | 3 | 4 | 2 | 0 | 0 | 0 | 0 | 0 | 2 | 2 | 0 |

TABLE 3 (continued)

| Compound of Ex. No. | Soil drench 10/kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 13 | 4 | 2 | 3 | 5 | 5 | 4 | 6 | 7 | 5 | 0 | 0 | 4 | 3 | 2 | 2 | 6 | 5 | 6 | 2 | 6 | 5 | 3 | 6 | 9 | 7 |
| | | | | | | | | | 1 | 0 | 0 | 0 | 0 | 1 | 1 | 4 | 5 | 0 | 0 | 0 | 2 | 0 | 1 | 6 | 0 |
| 14 | 2 | 0 | 0 | 4 | 6 | 6 | 7 | 4 | 5 | 0 | 0 | 4 | 0 | 4 | 4 | 6 | 3 | 0 | 0 | 2 | 2 | 4 | 4 | 3 | 0 |
| | | | | | | | | | 1 | 0 | 0 | 0 | 0 | 4 | 2 | 4 | 0 | 0 | 0 | 0 | 0 | 0 | 2 | 1 | 0 |
| 15 | 3 | 1 | 0 | 0 | 0 | 3 | 4 | 4 | 5 | 0 | 0 | 0 | 0 | 0 | 0 | 0 | 0 | 0 | 0 | 1 | 0 | 0 | 6 | 1 | 0 |
| | | | | | | | | | 1 | 0 | 0 | 0 | 0 | 0 | 0 | 0 | 0 | 0 | 0 | 0 | 0 | 0 | 2 | 1 | 0 |
| 16 | 0 | 0 | 2 | 3 | 2 | 4 | 5 | 4 | 5 | 5 | 0 | 3 | 2 | 3 | 4 | 4 | 3 | 0 | 0 | 0 | 0 | 0 | 0 | 0 | 0 |
| | | | | | | | | | 1 | 2 | 0 | 2 | 2 | 1 | 2 | 4 | 3 | 0 | 0 | 0 | 0 | 0 | 0 | 0 | 0 |

TABLE 3 (continued)

| Compound of Ex. No. | Soil drench 10/kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 17 | 0 | 3 | 4 | 4 | 0 | 3 | 4 | 3 | 5 | 2 | 0 | 5 | 0 | 3 | 0 | 5 | 5 | 0 | 0 | 0 | 0 | 0 | 0 | 0 | 0 |
| | | | | | | | | | 1 | 1 | 0 | 2 | 0 | 2 | 0 | 5 | 4 | 0 | 0 | 0 | 0 | 0 | 0 | 0 | 0 |
| 18 | 0 | 2 | 3 | 4 | 6 | 3 | 5 | 0 | 5 | 3 | 2 | 0 | 2 | 4 | 4 | 4 | 3 | 0 | 0 | 0 | 0 | 0 | 0 | 0 | 0 |
| | | | | | | | | | 1 | 2 | 0 | 0 | 1 | 2 | 2 | 2 | 1 | 0 | 0 | 0 | 0 | 0 | 0 | 0 | 0 |
| 19 | 0 | 0 | 0 | 0 | 4 | 6 | 5 | 3 | 5 | 0 | 0 | 0 | 0 | 0 | 0 | 0 | 0 | 0 | 0 | 2 | 1 | 4 | 6 | 4 | 0 |
| | | | | | | | | | 1 | 0 | 0 | 0 | 0 | 0 | 0 | 0 | 0 | 0 | 0 | 0 | 0 | 0 | 1 | 2 | 0 |
| 20 | 6 | 3 | 4 | 4 | 0 | 3 | 6 | 7 | 5 | 2 | 0 | 2 | 0 | 0 | 0 | 6 | 4 | 7 | 3 | 7 | 3 | 0 | 7 | 8 | 4 |
| | | | | | | | | | 1 | 2 | 0 | 1 | 0 | 0 | 0 | 4 | 2 | 0 | 1 | 4 | 1 | 0 | 0 | 5 | 1 |

TABLE 3 (continued)

| Compound of Ex. No. | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | kg/ha | Mz | R | BG | O | L | M | SB | S | Mz | R | BG | O | L | M | SB | S |
| 21 | 0 | 0 | 0 | 0 | 4 | 3 | 5 | 4 | 5 | 0 | 0 | 1 | 1 | 1 | 3 | 4 | 6 | 0 | 0 | 0 | 0 | 0 | 6 | 5 | 0 |
| | | | | | | | | | 1 | 0 | 0 | 0 | 0 | 0 | 2 | 2 | 3 | 0 | 0 | 0 | 0 | 0 | 2 | 2 | 0 |
| 22 | 0 | 3 | 2 | 2 | 3 | 2 | 7 | 5 | 5 | 3 | 0 | 3 | 0 | 0 | 2 | 6 | 5 | 4 | 4 | 4 | 3 | 0 | 5 | 8 | 4 |
| | | | | | | | | | 1 | 2 | 0 | 3 | 0 | 0 | 1 | 4 | 4 | 0 | 2 | 1 | 1 | 0 | 2 | 5 | 1 |
| 23 | 0 | 0 | 0 | 2 | 3 | 6 | 7 | 4 | 5 | 0 | 0 | 0 | 0 | 0 | 0 | 0 | 0 | 0 | 0 | 0 | 0 | 0 | 5 | 4 | 0 |
| | | | | | | | | | 1 | 0 | 0 | 0 | 0 | 0 | 0 | 0 | 0 | 0 | 0 | 0 | 0 | 0 | 1 | 1 | 0 |
| 24 | 6 | 2 | 5 | 3 | 4 | 4 | 7 | 7 | 5 | 3 | 3 | 4 | 2 | 0 | 0 | 7 | 6 | 7 | 5 | 6 | 4 | 3 | 7 | 9 | 6 |
| | | | | | | | | | 1 | 1 | 1 | 2 | 1 | 0 | 0 | 5 | 4 | 4 | 2 | 2 | 1 | 0 | 2 | 5 | 3 |

TABLE 3 (continued)

| Compound of Ex. No. | Soil drench 10/kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 25 | 0 | 0 | 2 | 2 | 3 | 0 | 4 | 3 | 5 | 0 | 0 | 0 | 2 | 3 | 0 | 3 | 5 | 0 | 0 | 0 | 0 | 0 | 0 | 0 | 0 |
| | | | | | | | | | 1 | 0 | 0 | 0 | 0 | 0 | 0 | 2 | 2 | 0 | 0 | 0 | 0 | 0 | 0 | 0 | 0 |
| 26 | 0 | 3 | 4 | 2 | 0 | 0 | 7 | 6 | 5 | 0 | 3 | 3 | 1 | 1 | 0 | 6 | 5 | 0 | 2 | 4 | 3 | 2 | 4 | 7 | 5 |
| | | | | | | | | | 1 | 0 | 0 | 2 | 0 | 0 | 0 | 4 | 5 | 0 | 1 | 1 | 1 | 1 | 2 | 4 | 0 |
| 27 | 0 | 0 | 0 | 0 | 0 | 0 | 6 | 6 | 5 | 0 | 0 | 2 | 2 | 2 | 3 | 4 | 4 | 0 | 0 | 0 | 0 | 0 | 0 | 5 | 5 |
| | | | | | | | | | 1 | 0 | 0 | 0 | 1 | 0 | 3 | 2 | 3 | 0 | 0 | 0 | 0 | 0 | 0 | 2 | 1 |
| 28 | 4 | 3 | 3 | 3 | 2 | 4 | 5 | 5 | 5 | 0 | 0 | 2 | 0 | 0 | 2 | 5 | 3 | 6 | 6 | 7 | 4 | 1 | 8 | 9 | 5 |
| | | | | | | | | | 1 | 0 | 0 | 1 | 0 | 0 | 2 | 3 | 2 | 0 | 2 | 0 | 0 | 0 | 0 | 4 | 0 |

TABLE 3 (continued)

| Compound of Ex. No. | Soil drench 10/kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 29 | 0 | 0 | 0 | 0 | 0 | 7 | 7 | 2 | 5 | 0 | 0 | 0 | 0 | 0 | 4 | 6 | 5 | 0 | 0 | 2 | 0 | 0 | 6 | 4 | 0 |
| | | | | | | | | | 1 | 0 | 0 | 0 | 0 | 0 | 2 | 4 | 2 | 0 | 0 | 0 | 0 | 0 | 1 | 2 | 0 |
| 30 | 0 | 0 | 0 | 1 | 1 | 2 | 5 | 4 | 5 | 0 | 0 | 0 | 0 | 0 | 0 | 0 | 0 | 0 | 0 | 0 | 0 | 2 | 3 | 5 | 1 |
| | | | | | | | | | 1 | 0 | 0 | 0 | 0 | 0 | 0 | 0 | 0 | 0 | 0 | 0 | 0 | 0 | 1 | 2 | 0 |
| 31 | 6 | 5 | 5 | 5 | 3 | 4 | 7 | 6 | 5 | 2 | 2 | 2 | 2 | 0 | 0 | 7 | 4 | 6 | 5 | 6 | 5 | 0 | 3 | 8 | 6 |
| | | | | | | | | | 1 | 1 | 1 | 1 | 1 | 0 | 0 | 5 | 3 | 0 | 1 | 2 | 3 | 0 | 2 | 6 | 3 |
| 32 | 4 | 2 | 3 | 3 | 0 | 0 | 5 | 4 | 5 | 0 | 0 | 0 | 0 | 0 | 0 | 6 | 4 | 0 | 0 | 0 | 0 | 0 | 0 | 0 | 0 |
| | | | | | | | | | 1 | 0 | 0 | 0 | 0 | 0 | 0 | 3 | 3 | 0 | 0 | 0 | 0 | 0 | 0 | 0 | 0 |

TABLE 3 (continued)

| Compound of | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 33 | 7 | 5 | 5 | 4 | 4 | 4 | 7 | 4 | 5 | 6 | 4 | 5 | 4 | 4 | 7 | 7 | 6 | 6 | 6 | 7 | 4 | 4 | 7 | 9 | 0 |
| | | | | | | | | | 1 | 2 | 2 | 4 | 2 | 4 | 7 | 7 | 5 | 5 | 4 | 5 | 4 | 2 | 5 | 7 | 0 |
| 34 | 3 | 2 | 2 | 0 | 0 | 4 | 7 | 3 | 5 | 5 | 3 | 3 | 3 | 3 | 6 | 7 | 7 | 0 | 0 | 0 | 2 | 0 | 5 | 6 | 0 |
| | | | | | | | | | 1 | 3 | 0 | 2 | 1 | 2 | 4 | 6 | 5 | 0 | 0 | 0 | 0 | 0 | 3 | 4 | 0 |
| 35 | 0 | 0 | 0 | 0 | 0 | 0 | 0 | 0 | 5 | 3 | 0 | 0 | 2 | 5 | 5 | 6 | 3 | 0 | 0 | 0 | 0 | 0 | 0 | 0 | 0 |
| | | | | | | | | | 1 | 2 | 0 | 0 | 1 | 2 | 3 | 4 | 3 | 0 | 0 | 0 | 0 | 0 | 0 | 0 | 0 |
| 37 | 3 | 3 | 4 | 4 | 0 | 2 | 6 | 6 | 5 | 0 | 0 | 0 | 0 | 1 | 1 | 4 | 3 | 5 | 2 | 7 | 0 | 0 | 0 | 9 | 4 |
| | | | | | | | | | 1 | 0 | 0 | 0 | 0 | 0 | 0 | 3 | 3 | 1 | 0 | 4 | 0 | 0 | 0 | 8 | 0 |

TABLE 3 (continued)

| Compound of Ex. No. | Soil drench 10/kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 38 | 3 | 4 | 2 | 3 | 0 | 5 | 5 | 2 | 5 | 2 | 0 | 1 | 1 | 2 | 4 | 5 | 4 | 5 | 2 | 3 | 2 | 3 | 5 | 9 | 2 |
| | | | | | | | | | 1 | 0 | 0 | 0 | 0 | 0 | 2 | 3 | 3 | 3 | 0 | 0 | 0 | 0 | 2 | 4 | 0 |
| 39 | 5 | 4 | 4 | 1 | 0 | 5 | 5 | 2 | 5 | 3 | 0 | 2 | 0 | 2 | 3 | 5 | 4 | 6 | 5 | 4 | 0 | 2 | 6 | 8 | 2 |
| | | | | | | | | | 1 | 0 | 0 | 0 | 0 | 1 | 2 | 5 | 2 | 3 | 2 | 1 | 0 | 0 | 2 | 6 | 0 |
| 40 | 6 | 5 | 6 | 4 | 3 | 6 | 6 | 4 | 5 | 4 | 2 | 3 | 2 | 3 | 5 | 6 | 4 | 7 | 6 | 7 | 3 | 2 | 7 | 9 | 5 |
| | | | | | | | | | 1 | 2 | 0 | 1 | 0 | 1 | 3 | 5 | 3 | 5 | 3 | 3 | 0 | 0 | 2 | 7 | 2 |
| 41 | 2 | 3 | 3 | 2 | 0 | 4 | 5 | 2 | 5 | 1 | 0 | 1 | 1 | 2 | 4 | 6 | 3 | 5 | 4 | 4 | 0 | 0 | 6 | 8 | 0 |
| | | | | | | | | | 1 | 0 | 0 | 0 | 0 | 1 | 3 | 6 | 3 | 2 | 1 | 1 | 0 | 0 | 3 | 7 | C |

TABLE 3 (continued)

| Compound of | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 42 | 4 | 3 | 3 | 2 | 0 | 4 | 5 | 3 | 5 | 1 | 0 | 0 | 0 | 2 | 4 | 5 | 4 | 6 | 7 | 6 | 2 | 2 | 6 | 9 | 3 |
| | | | | | | | | | 1 | 0 | 0 | 0 | 0 | 0 | 3 | 4 | 3 | 4 | 3 | 1 | 0 | 0 | 2 | 8 | 0 |
| 43 | 2 | 0 | 0 | 2 | 1 | 1 | 5 | 4 | 5 | 0 | 0 | 1 | 2 | 3 | 1 | 5 | 4 | 0 | 0 | 0 | 0 | 0 | 0 | 8 | 3 |
| | | | | | | | | | 1 | 0 | 0 | 0 | 0 | 1 | 0 | 2 | 1 | 0 | 0 | 0 | 0 | 0 | 0 | 0 | 0 |
| 44 | 5 | 3 | 4 | 3 | 2 | 3 | 5 | 4 | 5 | 3 | 0 | 4 | 3 | 5 | 5 | 5 | 5 | 5 | 4 | 5 | 3 | 0 | 6 | 8 | 0 |
| | | | | | | | | | 1 | 1 | 0 | 2 | 2 | 2 | 4 | 4 | 4 | 2 | 2 | 3 | 1 | 0 | 3 | 6 | 0 |
| 45 | 3 | 3 | 3 | 3 | 2 | 2 | 5 | 0 | 5 | 3 | 2 | 2 | 1 | 4 | 5 | 6 | 3 | 5 | 2 | 3 | 2 | 0 | 5 | 8 | 0 |
| | | | | | | | | | 1 | 3 | 1 | 1 | 0 | 3 | 4 | 5 | 3 | 2 | 0 | 1 | 1 | 0 | 2 | 5 | 0 |

TABLE 3 (continued)

| Compound of Ex. No. | Soil drench 10/kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 46 | 5 | 3 | 4 | 3 | 0 | 4 | 6 | 5 | 5 | 4 | 0 | 2 | 2 | 1 | 3 | 5 | 4 | 6 | 1 | 5 | 2 | 0 | 5 | 8 | 1 |
| | | | | | | | | | 1 | 3 | 0 | 1 | 1 | 1 | 2 | 4 | 3 | 2 | 0 | 3 | 1 | 0 | 1 | 2 | 0 |
| 47 | 4 | 4 | 5 | 4 | 0 | 0 | 4 | 1 | 5 | 2 | 0 | 1 | 1 | 0 | 3 | 4 | 4 | 5 | 2 | 4 | 0 | 0 | 0 | 7 | 0 |
| | | | | | | | | | 1 | 1 | 0 | 1 | 1 | 0 | 2 | 3 | 3 | 1 | 1 | 2 | 0 | 0 | 0 | 4 | 0 |
| 48 | 0 | 0 | 0 | 1 | 0 | 0 | 6 | 0 | 5 | 3 | 1 | 0 | 0 | 3 | 4 | 5 | 3 | 0 | 0 | 0 | 0 | 0 | 0 | 7 | 0 |
| | | | | | | | | | 1 | 1 | 1 | 0 | 0 | 2 | 4 | 5 | 3 | 0 | 0 | 0 | 0 | 0 | 0 | 6 | 0 |
| 49 | 4 | 3 | 3 | 2 | 0 | 0 | 5 | 0 | 5 | 2 | 2 | 2 | 1 | 4 | 4 | 5 | 4 | 3 | 4 | 3 | 1 | 0 | 4 | 8 | 0 |
| | | | | | | | | | 1 | 1 | 1 | 2 | 1 | 3 | 4 | 4 | 4 | 2 | 3 | 3 | 0 | 0 | 3 | 7 | 0 |

TABLE 3 (continued)

| Compound of | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 50 | 6 | 3 | 4 | 2 | 2 | 2 | 7 | 5 | 5 | 0 | 0 | 0 | 0 | 0 | 0 | 5 | 4 | 5 | 3 | 6 | 2 | 2 | 0 | 8 | 4 |
| | | | | | | | | | 1 | 0 | 0 | 0 | 0 | 0 | 0 | 4 | 3 | 0 | 0 | 3 | 1 | 1 | 0 | 7 | 0 |
| 51 | 5 | 3 | 3 | 2 | 0 | 0 | 6 | 2 | 5 | 3 | 1 | 1 | 1 | 0 | 0 | 4 | 4 | 3 | 1 | 2 | 1 | 0 | 0 | 7 | 2 |
| | | | | | | | | | 1 | 2 | 1 | 1 | 0 | 0 | 0 | 3 | 2 | 1 | 1 | 1 | 0 | 0 | 0 | 4 | 0 |
| 52 | 5 | 4 | 4 | 1 | 0 | 3 | 6 | 4 | 5 | 2 | 1 | 4 | 0 | 4 | 5 | 6 | 5 | 5 | 4 | 5 | 1 | 0 | 6 | 8 | 1 |
| | | | | | | | | | 1 | 2 | 1 | 2 | 0 | 3 | 4 | 4 | 3 | 3 | 4 | 3 | 0 | 0 | 2 | 6 | 0 |
| 53 | 7 | 6 | 6 | 5 | 3 | 5 | 7 | 6 | 5 | 4 | 4 | 5 | 2 | 3 | 5 | 6 | 6 | 5 | 5 | 7 | 4 | 3 | 8 | 9 | 2 |
| | | | | | | | | | 1 | 2 | 2 | 3 | 1 | 2 | 4 | 5 | 6 | 3 | 5 | 4 | 1 | 1 | 6 | 9 | 1 |

TABLE 3 (continued)

| Compound of | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 54 | 4 | 3 | 0 | 1 | 0 | 0 | 5 | 4 | 5 | 3 | 0 | 1 | 1 | 4 | 4 | 4 | 6 | 0 | 0 | 0 | 0 | 0 | 0 | 6 | 1 |
| | | | | | | | | | 1 | 2 | 0 | 1 | 1 | 2 | 2 | 4 | 5 | 0 | 0 | 0 | 0 | 0 | 0 | 6 | 1 |
| 55 | 0 | 0 | 0 | 0 | 0 | 0 | 0 | 0 | 5 | 3 | 1 | 5 | 1 | 3 | 5 | 8 | 6 | 3 | 0 | 4 | 0 | 0 | 0 | 8 | 1 |
| | | | | | | | | | 1 | 1 | 1 | 2 | 0 | 1 | 2 | 5 | 3 | 0 | 0 | 0 | 0 | 0 | 0 | 2 | 0 |
| 56 | 6 | 5 | 6 | 3 | 3 | 5 | 6 | 5 | 5 | 2 | 2 | 4 | 1 | 4 | 6 | 7 | 6 | 5 | 4 | 6 | 0 | 0 | 4 | 8 | 2 |
| | | | | | | | | | 1 | 2 | 1 | 2 | 0 | 2 | 4 | 5 | 5 | 3 | 1 | 4 | 0 | 0 | 4 | 8 | 1 |
| 57 | 5 | 3 | 4 | 2 | 2 | 0 | 5 | 5 | 5 | 2 | 1 | 5 | 3 | 5 | 5 | 6 | 5 | 2 | 0 | 4 | 0 | 0 | 0 | 8 | 0 |
| | | | | | | | | | 1 | 0 | 0 | 3 | 1 | 3 | 3 | 4 | 3 | 1 | 0 | 2 | 0 | 0 | 0 | 3 | 0 |

TABLE 3 (continued)

| Compound of | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 58 | 0 | 0 | 0 | 0 | 0 | 0 | 0 | 0 | 5 | 3 | 2 | 4 | 3 | 4 | 7 | 6 | 7 | 0 | 0 | 3 | 0 | 0 | 0 | 8 | 1 |
| | | | | | | | | | 1 | 2 | 1 | 3 | 0 | 2 | 4 | 4 | 4 | 0 | 0 | 0 | 0 | 0 | 0 | 6 | 0 |
| 59 | 6 | 4 | 5 | 1 | 2 | 4 | 6 | 3 | 5 | 4 | 2 | 4 | 2 | 2 | 3 | 5 | 4 | 4 | 2 | 5 | 2 | 2 | 5 | 9 | 3 |
| | | | | | | | | | 1 | 2 | 0 | 3 | 1 | 1 | 2 | 5 | 3 | 2 | 0 | 2 | 1 | 0 | 4 | 7 | 0 |
| 60 | 3 | 2 | 3 | 2 | 0 | 0 | 4 | 2 | 5 | 2 | 2 | 0 | 0 | 0 | 4 | 6 | 4 | 1 | 0 | 3 | 0 | 0 | 5 | 7 | 0 |
| | | | | | | | | | 1 | 2 | 1 | 0 | 0 | 0 | 2 | 5 | 2 | 0 | 0 | 1 | 0 | 0 | 2 | 6 | 0 |
| 61 | 5 | 4 | 5 | 3 | 4 | 5 | 6 | 5 | 5 | 4 | 1 | 3 | 2 | 0 | 4 | 6 | 4 | 6 | 4 | 6 | 2 | 2 | 5 | 9 | 4 |
| | | | | | | | | | 1 | 3 | 0 | 0 | 0 | 0 | 2 | 5 | 3 | 3 | 2 | 3 | 1 | 0 | 1 | 8 | 0 |

TABLE 3 (continued)

| Compound of Ex. No. | Soil drench 10/kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 62 | 4 | 3 | 4 | 2 | 0 | 2 | 4 | 2 | 5 | 6 | 3 | 7 | 4 | 3 | 3 | 6 | 7 | 2 | 1 | 4 | 2 | 0 | 3 | 8 | 1 |
| | | | | | | | | | 1 | 3 | 1 | 5 | 1 | 2 | 2 | 6 | 6 | 0 | 0 | 0 | 1 | 1 | 3 | 8 | 0 |
| 63 | 2 | 2 | 3 | 1 | 0 | 1 | 4 | 0 | 5 | 4 | 1 | 6 | 2 | 5 | 5 | 7 | 6 | 1 | 0 | 1 | 1 | 2 | 7 | 9 | 0 |
| | | | | | | | | | 1 | 3 | 1 | 4 | 2 | 4 | 4 | 7 | 5 | 0 | 0 | 0 | 1 | 1 | 3 | 8 | 0 |
| 64 | 4 | 2 | 5 | 3 | 2 | 3 | 6 | 4 | 5 | 3 | 0 | 4 | 0 | 2 | 4 | 5 | 5 | 2 | 0 | 4 | 2 | 0 | 6 | 8 | 0 |
| | | | | | | | | | 1 | 2 | 0 | 2 | 0 | 1 | 3 | 4 | 4 | 1 | 0 | 2 | 2 | 0 | 5 | 8 | 0 |
| 65 | 1 | 0 | 1 | 0 | 0 | 0 | 7 | 3 | 5 | 0 | 0 | 0 | 0 | 0 | 2 | 5 | 4 | 0 | 0 | 0 | 0 | 0 | 0 | 0 | 0 |
| | | | | | | | | | 1 | 0 | 0 | 0 | 0 | 0 | 0 | 3 | 2 | 0 | 0 | 0 | 0 | 0 | 0 | 0 | 0 |

TABLE 3 (continued)

| Compound of | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 66 | 0 | 0 | 0 | 0 | 0 | 0 | 0 | 0 | 5 | 0 | 0 | 0 | 0 | 0 | 2 | 2 | 3 | 0 | 0 | 0 | 0 | 0 | 0 | 5 | 0 |
| | | | | | | | | | 1 | | | | | | | | 1 | | | | | | | | |
| 67 | 2 | 0 | 0 | 0 | 0 | 0 | 3 | 0 | 5 | 2 | 0 | 0 | 0 | 0 | 1 | 4 | 2 | 3 | 0 | 2 | 3 | 0 | 0 | 7 | 0 |
| | | | | | | | | | 1 | 0 | 0 | 0 | 0 | 0 | 1 | 4 | 1 | 0 | 0 | 0 | 0 | 0 | 0 | 5 | 0 |
| 69 | 0 | 0 | 0 | 0 | 0 | 0 | 0 | 0 | 5 | 0 | 0 | 0 | 0 | 4 | 6 | 5 | 4 | 0 | 0 | 0 | 0 | 0 | 0 | 0 | 0 |
| | | | | | | | | | 1 | 0 | 0 | 0 | 0 | 3 | 2 | 2 | 2 | 0 | 0 | 0 | 0 | 0 | 0 | 0 | 0 |
| 71 | 0 | 0 | 0 | 0 | 0 | 0 | 3 | 1 | 5 | 1 | 0 | 0 | 1 | 2 | 0 | 4 | 4 | 0 | 0 | 0 | 0 | 0 | 0 | 0 | 0 |
| | | | | | | | | | 1 | 1 | 0 | 0 | 0 | 1 | 0 | 3 | 3 | 0 | 0 | 0 | 0 | 0 | 0 | 0 | 0 |

TABLE 3 (continued)

| Compound of Ex. No. | Soil drench 10/kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 72 | 0 | 0 | 2 | 1 | 0 | 2 | 3 | 1 | 5 | 3 | 1 | 1 | 0 | 0 | 1 | 0 | 4 | 0 | 0 | 0 | 0 | 0 | 0 | 0 | 0 |
| | | | | | | | | | 1 | 1 | 0 | 0 | 0 | 0 | 0 | 0 | 2 | 0 | 0 | 0 | 0 | 0 | 0 | 0 | 0 |
| 73 | 0 | 2 | 0 | 2 | 0 | 0 | 3 | 0 | 5 | 4 | 1 | 1 | 0 | 3 | 4 | 5 | 3 | 0 | 0 | 0 | 0 | 0 | 0 | 0 | 0 |
| | | | | | | | | | 1 | 2 | 0 | 0 | 0 | 1 | 1 | 1 | 3 | 0 | 0 | 0 | 0 | 0 | 0 | 0 | 0 |
| 74 | 0 | 0 | 0 | 0 | 0 | 0 | 0 | 0 | 5 | 0 | 0 | 0 | 0 | 0 | 0 | 0 | 1 | 0 | 0 | 0 | 0 | 0 | 0 | 0 | 0 |
| | | | | | | | | | 1 | 0 | 0 | 0 | 0 | 0 | 0 | 0 | 1 | 0 | 0 | 0 | 0 | 0 | 0 | 0 | 0 |
| 75 | 8 | 5 | 7 | 7 | 4 | 7 | 8 | 7 | 5 | 4 | 2 | 4 | 0 | 3 | 5 | 6 | 7 | 8 | 5 | 6 | 5 | 0 | 6 | 9 | 6 |
| | | | | | | | | | 1 | 3 | 1 | 1 | 0 | 0 | 2 | 4 | 4 | 3 | 1 | 3 | 2 | 0 | 2 | 8 | 3 |

TABLE 3 (continued)

| Compound of | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 76 | 6 | 3 | 6 | 5 | 2 | 7 | 7 | 6 | 5 | 4 | 1 | 4 | 4 | 3 | 3 | 5 | 6 | 7 | 5 | 6 | 3 | 1 | 5 | 9 | 1 |
| | | | | | | | | | 1 | 2 | 1 | 1 | 0 | 1 | 1 | 4 | 3 | 5 | 3 | 3 | 3 | 0 | 1 | 8 | 0 |
| 77 | 4 | 0 | 3 | 5 | 0 | 3 | 5 | 4 | 5 | 3 | 1 | 2 | 1 | 2 | 3 | 4 | 4 | 6 | 1 | 5 | 5 | 0 | 3 | 8 | 4 |
| | | | | | | | | | 1 | 1 | 0 | 0 | 0 | 0 | 0 | 2 | 2 | 1 | 0 | 0 | 2 | 0 | 0 | 7 | 0 |
| 78 | 4 | 1 | 4 | 4 | 0 | 0 | 0 | 3 | 5 | 2 | 0 | 0 | 1 | 1 | 0 | 5 | 2 | 0 | 0 | 4 | 4 | 0 | 2 | 8 | 0 |
| | | | | | | | | | 1 | 1 | 0 | 0 | 1 | 1 | 0 | 4 | 2 | 0 | 0 | 0 | 2 | 0 | 0 | 6 | 0 |
| 79 | 5 | 2 | 3 | 3 | 0 | 0 | 4 | 2 | 5 | 4 | 0 | 3 | 1 | 3 | 4 | 6 | 3 | 0 | 0 | 3 | 3 | 0 | 0 | 8 | 0 |
| | | | | | | | | | 1 | 2 | 0 | 2 | 1 | 2 | 1 | 4 | 2 | 0 | 0 | 0 | 1 | 0 | 0 | 5 | 0 |

TABLE 3 (continued)

| Compound of Ex. No. | Soil drench 10/kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 80 | 7 | 3 | 5 | 7 | 3 | 5 | 6 | 6 | 5 | 3 | 0 | 0 | 0 | 2 | 4 | 6 | 3 | 7 | 0 | 6 | 6 | 0 | 3 | 9 | 2 |
| | | | | | | | | | 1 | 0 | 0 | 0 | 0 | 0 | 0 | 5 | 3 | 2 | 0 | 0 | 2 | 0 | 0 | 8 | 0 |
| 81 | 6 | 4 | 6 | 5 | 0 | 6 | 6 | 3 | 5 | 5 | 3 | 4 | 4 | 4 | 4 | 6 | 6 | 7 | 6 | 5 | 4 | 0 | 4 | 9 | 0 |
| | | | | | | | | | 1 | 3 | 1 | 2 | 2 | 3 | 3 | 4 | 4 | 6 | 3 | 3 | 2 | 0 | 1 | 8 | 0 |
| 82 | 6 | 2 | 4 | 4 | 1 | 3 | 6 | 2 | 5 | 3 | 0 | 3 | 3 | 3 | 5 | 6 | 4 | 6 | 2 | 5 | 6 | 5 | 7 | 9 | 0 |
| | | | | | | | | | 1 | 2 | 0 | 2 | 1 | 2 | 3 | 4 | 3 | 4 | 1 | 3 | 3 | 1 | 4 | 8 | 0 |
| 83 | 2 | 0 | 0 | 0 | 0 | 0 | 0 | 0 | 5 | 0 | 0 | 0 | 0 | 0 | 0 | 0 | 0 | 0 | 0 | 0 | 0 | 0 | 0 | 7 | 0 |
| | | | | | | | | | 1 | 0 | 0 | 0 | 0 | 0 | 0 | 0 | 0 | 0 | 0 | 0 | 0 | 0 | 0 | 4 | 0 |

TABLE 3 (continued)

| Compound of | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 84 | 6 | 2 | 3 | 5 | 0 | 0 | 4 | 2 | 5 | 1 | 0 | 2 | 1 | 2 | 3 | 6 | 3 | 3 | 0 | 4 | 3 | 2 | 5 | 9 | 0 |
| | | | | | | | | | 1 | 1 | 0 | 1 | 1 | 1 | 2 | 6 | 3 | 2 | 0 | 2 | 2 | 0 | 0 | 3 | 0 |

## Claims

1. A herbicidal composition which comprises a carrier and, as active ingredient, a compound of the general formula I or a salt thereof:

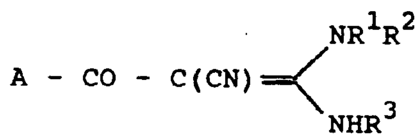

(I)

in which A represents a phenyl group optionally substituted by one or more of the same or different substituents selected from halogen atoms and nitro, cyano, amino, alkylamino, dialkylamino, hydroxy, phenoxy, alkyl, haloalkyl, alkylthio, alkylsulphenyl, alkoxycarbonyl, carboxy and alkoxy groups and each of $R^1$, $R^2$, and $R^3$ independently represents a hydrogen atom, a cyclopropyl group, or an alkyl, alkenyl, alkynyl, alkoxy, alkenyloxy or alkynyloxy group optionally substituted by one or more the same or different substituents selected from halogen atoms and alkoxy, alkylthio, amino, alkylamino and dialkylamino groups; the total number of carbon atoms present in each of $R^1$, $R^2$, or $R^3$ being not more than 6; or $R^1$ has one of the meanings given above, and $R^2$ and $R^3$ together represent a dimethylene or trimethylene group.

2. A composition as claimed in claim 1, in which A represents a phenyl group mono-substituted or disubstituted by chlorine and/or fluorine atoms.

3. A composition as claimed in claim 1 or claim 2, in which $R^1$ represents a hydrogen atom.

4. A composition as claimed in any one of claims 1 to 3, in which each of $R^2$ and $R^3$ independently represents a cyclopropyl group or an alkyl, alenyl, alkynyl, alkoxy, alkenyloxy or alkynyloxy group having up to 4 carbon atoms, optionally substituted by a halogen, methoxy, methylamino or dimethylamino moiety, or $R^2$ and $R^3$ together represent a dimethylene group.

5. A composition as claimed in claim 4, in which each of $R^2$ and $R^3$ independently represents an unsubstituted alkyl group having 2 or 3 carbon atoms.

6. A composition as claimed in any one of claims 1 to 5, which comprises at least two carriers, at least one of which is a surface-active agent.

7. A compound of the general formula I as defined in any one of claims 1 to 7, or a salt thereof, with the provisos that (i) if the phenyl group A is mono-substituted in the 4-position by chlorine, bromine or methoxy, or di-substituted in the 3,4-positions by chlorine, and $R^2$ and $R^3$ together represent dimethylene, then $R^1$ is other than hydrogen; (ii) if the phenyl group A is unsubstituted or mono-substituted in the 4-position by chlorine or methyl, and $R^2$ and $R^3$ together represent trimethylene, then $R^1$ is other than methyl; and (iii) if $R^1$ is hydrogen and $R^2$ and $R^3$ are the same and are hydrogen, methyl or ethyl or if $R^1$ is hydrogen or methyl and $R^2$ and $R^3$ together are dimethylene, then the phenyl group A must carry at least one substituent.

8. A process for the preparation of a compound as claimed in claim 7, which comprises reacting a compound of the general formula

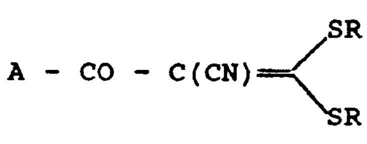

(II)

in which each R represents an alkyl group and A has the meaning given in claim 7, with a compound of the general formula $NHR^1R^2$, optionally followed by reaction with a compound of the general formula $NH_2R^3$; or with a compound of the general formula $NHR^1(CH_2)_nNH_2$ in which n is 2 or 3.

9. A method of combating undesired plant growth at a locus, which comprises treating the locus with a composition as claimed in any one of claims 1 to 6, or a compound as claimed in claim 7.

10. The use of a composition as claimed in any one of claims 1 to 6, or a compound as claimed in claim 7, as a herbicide.

**Patentansprüche**

1. Herbizides Mittel, umfassend einen Träger und, als Wirkstoff, eine Verbindung der allgemeinen Formel I oder ein Salz davon

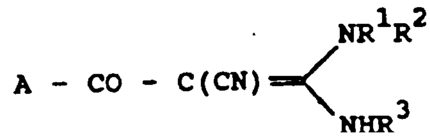

(I)

wobei A eine Phenylgruppe bedeutet, die gegebenenfalls substituiert ist durch einen oder mehrere gleiche oder verschiedene Substituienten, ausgewählt aus Halogenatomen und Nitro-, Cyano-, Amino-, Alkylamino-, Dialkylamino-, Hydroxy-, Phenoxy-, Alkyl-, Halogenalkyl-, Alkylthio-, Alkylsulphenyl-, Alkoxycarbonyl-, Carboxy- und Alkoxygruppen, und jeder der Reste $R^1$, $R^2$ und $R^3$ unabhängig voneinander ein Wasserstoffatom, eine Cyclopropylgruppe oder eine Alkyl-, Alkenyl-, Alkinyl-, Alkoxy-, Alkenyloxy- oder Alkinyloxygruppe bedeutet, gegebenenfalls substituiert durch einen oder mehrere gleiche oder verschiedene Substituenten, ausgewählt aus Halogenatomen und Alkoxy-, Alkylthio-, Amino-, Alkylamino- und Dialkylaminogruppen, wobei die Gesamtzahl von Kohlenstoffatomen in jedem der Reste $R^1$, $R^2$ oder $R^3$ nicht mehr als 6 ist; oder $R^1$ eine der oben angegebenen Bedeutungen hat und $R^2$ und $R^3$ zusammen eine Dimethylen- oder Trimethylengruppe bilden.

2. Mittel nach Anspruch 1, bei dem A eine Phenylgruppe bedeutet, die mono- oder disubstituiert ist durch Chlor- und/oder Fluoratome.

3. Mittel nach Anspruch 1 oder 2, wobei $R^1$ ein Wasserstoffatom bedeutet.

4. Mittel nach einem der Ansprüche 1 bis 3, wobei jeder der Reste $R^2$ und $R^3$ unabhängig voneinander eine Cyclopropylgruppe oder eine Alkyl-, Alkenyl-, Alkinyl-, Alkoxy-, Alkenyloxy- oder Alkinyloxygruppe mit bis zu 4 Kohlenstoffatomen bedeutet, gegebenenfalls substituiert durch eine Halogen-, Methoxy-, Methylamino- oder Dimethylaminogruppe, oder $R^2$ und $R^3$ zusammen eine Dimethylengruppe bilden.

5. Mittel nach Anspruch 4, wobei jeder der Reste $R^2$ und $R^3$ unabhängig voneinander eine unsubstituierte Alkylgruppe mit 2 bis 3 Kohlenstoffatomen bedeutet.

6. Mittel nach einem der Ansprüche 1 bis 5, umfassend zumindest zwei Träger, von denen zumindest einer ein grenzflächenaktives Mittel ist.

7. Verbindung der allgemeinen Formel I, wie in einem der Ansprüche 1 bis 7 definiert, oder ein Salz davon, mit den Maßgaben, daß (i) wenn die Phenylgruppe A in 4-Stellung durch Chlor, Brom oder Methoxy monosubstituiert oder in 3- und 4-Stellung durch Chlor disubstituiert ist und $R^2$ und $R^3$ zusammen eine Dimethylengruppe bedeuten, $R^1$ kein Wasserstoff ist; (ii) wenn die Phenylgruppe A unsubstituiert oder in 4-Stellung durch Chlor oder Methyl monosubstituiert ist und $R^2$ und $R^3$ zusammen eine Trimethylengruppe bedeuten, $R^1$ nicht Methyl ist, und (iii) wenn $R^1$ Wasserstoff ist und $R^2$ und $R^3$ gleich sind und Wasserstoff, Methyl oder Ethyl bedeuten oder, wenn $R^1$ Wasserstoff oder Methyl bedeutet und $R^2$ und $R^3$ zusammen eine Dimethylengruppe bilden, die Phenylgruppe A zumindest einen Substituenten enthalten muß.

8. Verfahren zur Herstellung einer Verbindung nach Anspruch 7, umfassend die Umsetzung einer Verbindung der allgemeinen Formel

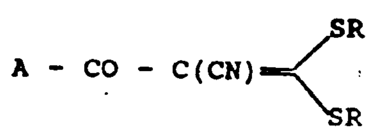

(II)

in der R eine Alkylgruppe bedeutet und A die in Anspruch 7 angegebene Bedeutung hat, mit einer Verbindung der allgemeinen Formel $NHR^1R^2$ und gegebenenfalls anschließende Umsetzung mit einer Verbindung der allgemeinen Formel $NH_2R^3$; oder mit einer Verbindung der allgemeinen Formel $NHR^1(CH_2)_nNH_2$, in der n 2 oder 3 ist.

9. Verfahren zur Bekämpfung von unerwünschtem Pflanzenwachstum an einer Stelle, umfassend die Behandlung der Stelle mit einem Mittel nach einem der Ansprüche 1 bis 6 oder einer Verbindung nach Anspruch 7.

10. Verwendung eines Mittels nach einem der Ansprüche 1 bis 6 oder einer Verbindung nach Anspruch 7 als Herbizid.

## Revendications

1. Composition herbicide, comprenant un véhicule et, comme ingrédient actif, un composé de formule générale I ou un sel de celui-ci :

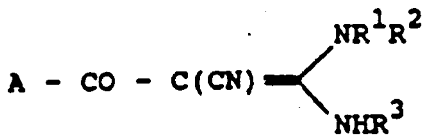

(I)

dans laquelle A représente un groupe phényle éventuellement substitué avec au moins un substituant identique ou différent choisi parmi les atomes d'halogène et les groupes nitro, cyano, amino, alkylamino, dialkylamino, hydroxy, phénoxy, alkyle, haloalkyle, alkylthio, alkylsulfonyle, alkoxycarbonyle, carboxy et alkoxy, et chaque groupe $R^1$, $R^2$, $R^3$ représente indépendamment un atome d'hydrogène, un groupe cyclopropyle, ou un groupe alkyle, alkényle, alkynyle, alkoxy, alkényloxy ou alkylnyloxy éventuellement substitué avec un ou plusieurs substituants identiques ou différents choisis parmi les atomes d'halogène et les groupes alkoxy, alkylthio, amino, alkylamino et dialkylamino ; le nombre total d'atomes de carbone présents dans chacun des groupes $R^1$, $R^2$ et $R^3$ n'étant pas supérieur à 6 ; ou le groupe $R^1$ a l'une des définitions mentionnées ci-dessus, et $R^2$ ainsi que $R^3$ forment ensemble un groupe diméthylène ou triméthylène.

2. Composition selon la revendication 1, dans laquelle A représente un groupe phényle mono-substitué ou di-substitué avec des atomes de chlore et/ou de fluor.

3. Composition selon la revendication 1 ou 2, dans laquelle $R^1$ représente un atome d'hydrogène.

4. Composition selon l'une quelconque des revendications 1 à 3, dans laquelle chacun des groupes $R^2$ et $R^3$ représente de préférence, indépendamment un groupe cyclopropyle ou un groupe alkyle, alkényle, alkynyle, alkoxy, alkényloxy ou alkynyloxy comportant jusqu'à 4 atomes de carbone, éventuellement substitué avec un atome d'halogène ou un groupe méthoxy, méthylamino ou diméthylamino, ou les groupes $R^2$ et $R^3$ forment ensemble un groupe diméthylène.

5. Composition selon la revendication 4, dans laquelle chacun des groupes $R^2$ et $R^3$ représente indépendamment un groupe alkyle non substitué comportant 2 ou 3 atomes de carbone.

6. Composition selon l'une quelconque des revendications 1 à 5, contenant au moins deux véhicules, dont au moins un est un agent tensioactif.

7. Composé de formule générale I, tel que défini dans l'une quelconque des revendications 1 à 7, ou un sel de celui-ci, à condition (i) que si le groupe phényle A est mono-substitué en position 4 avec un atome de chlore, un atome de brome ou un groupe méthoxy, ou s'il est di-substitué aux positions 3 et 4 avec un atome de chlore, et si les groupes $R^2$ et $R^3$ forment ensemble un groupe diméthylène, alors $R^1$ est autre qu'un atome d'hydrogène ; (ii) si le groupe phényle A est non-substitué ou mono-substitué à la position 4 avec un atome de chlore ou un groupe méthyle, et si $R^2$ et $R^3$ forment ensemble un groupe triméthylène, alors $R^1$ est autre qu'un groupe méthyle ; et (iii) si $R^1$ représente un atome d'hydrogène, et si $R^2$ et $R^3$ sont identiques et représentent des atomes d'hydrogène, des groupes méthyle ou éthyle, ou si $R^1$ représente un atome d'hydrogène ou un groupe méthyle et si $R^2$ et $R^3$ forment ensemble un groupe diméthylène, le groupe phényle A doit alors comporter au moins un substituant.

8. Procédé de préparation d'un nouveau composé selon la revendication 7, dans lequel on fait réagir un composé de formule générale :

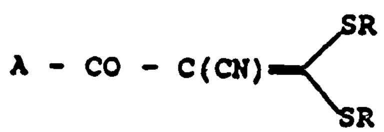

(II)

dans laquelle chaque groupe R représente un groupe alkyle, et A a la même définition que dans la revendication 7, avec un composé de formule générale $NHR^1R^2$, puis en effectuant éventuellement une réaction avec un composé de formule générale $NH_2R^3$ ; ou avec un composé de formule générale $NHR^1(CH_2)_nNH_2$ dans laquelle n est égal à 2 ou 3.

9. Procédé de lutte contre la croissance d'une plante non souhaitable dans une zone, selon lequel on traite la zone avec une composition définie dans l'une des revendications 1 à 6 ou un composé défini dans la revendication 7.

10. Utilisation comme herbicide d'une composition définie dans l'une quelconque des revendications 1 à 6, ou d'un composé défini dans la revendication 7.